## Europäisches Patentamt
## European Patent Office
## Office européen des brevets

(19)

(11) Veröffentlichungsnummer : **0 071 017**
**B1**

(12) **EUROPÄISCHE PATENTSCHRIFT**

(45) Veröffentlichungstag der Patentschrift :
07.09.88

(51) Int. Cl.⁴ : **A 61 B   6/00, A 61 B   6/02**

(21) Anmeldenummer : **82105528.2**

(22) Anmeldetag : **23.06.82**

(54) **Röntgenuntersuchungsgerät.**

(30) Priorität : **24.07.81 DE 3129307**

(43) Veröffentlichungstag der Anmeldung :
**09.02.83 Patentblatt 83/06**

(45) Bekanntmachung des Hinweises auf die Patenterteilung : **07.09.88 Patentblatt 88/36**

(84) Benannte Vertragsstaaten :
**FR GB**

(56) Entgegenhaltungen :
**CH-A-   269 812**

(73) Patentinhaber : **Siemens Aktiengesellschaft Berlin
und München
Wittelsbacherplatz 2
D-8000 München 2 (DE)**

(72) Erfinder : **Warden, Hans
Rimbograend 12
S-194 00 Upplands-Vaesby (SE)**

EP 0 071 017 B1

**Beschreibung**

Die Erfindung betrifft ein Röntgenuntersuchungsgerät mit einem um eine Achse schwenkbaren Stativ, das auf einer Seite der Achse mit einer Röntgenröhre und auf der anderen Seite mit einer Anordnung zum Verstellen eines Bildaufzeichnungsträgers entlang dem Stativ versehen ist.

Ein Röntgenuntersuchungsgerät dieser Art ist durch den Elema-Schönander-Prospekt « Mimer III » bekannt. Mit dem Gerät können Aufnahmen in jeder Winkellage des Statives mit dem Bildaufzeichnungsträger, z. B. einer Kassettenhalterung mit einer Kassette, senkrecht zum Röntgen-Zentralstrahl vorgenommen werden. Die Höhenverstellung der Kassettenhalterung erfolgt auf einer Säule, die in einer Achse an dem Stativ befestigt ist. Mit dem Gerät können ferner Tomographie-Aufnahmen gemacht werden, indem die stets parallel zu einem auf einem Untersuchungstisch liegenden Patienten ausgerichtete Kassettenhalterung beim Schwenken des Statives eine bogenförmige Bewegung beschreibt. Dies erfolgt, indem die Säule, auch in einer Schäglage des Statives, immer senkrecht zum Patienten steht. Wenn die Kassettenhalterung im Bereich der Achse der Säule angebracht ist, trifft der durch die Schwenk-Achse des Statives verlaufende Röntgenzentralstrahl die Mitte der Kassettenhalterung. Für eine einwandfreie Tomographieuntersuchung muss dabei der zu untersuchende Bereich des Patienten in Höhe der Schwenkachse liegen. Es besteht aber der Bedarf, den Objekt-Film-Abstand zu verringern, damit schärfere Röntgenaufnahmen erhalten werden. Dies wird erreicht, indem die Kassettenhalterung auf der Säule nach oben versetzt wird. Der Nachteil mit einer derartigen Verschiebung der Kassettenhalterung liegt darin, dass der Röntgenzentralstrahl bei einer Schräglage des Statives nicht mehr das Zentrum der Filmkassettenhalterung bzw. der Filmkassette trifft. Dies führt dazu, dass das durch eine Röntgenblende seitlich begrenzte Röntgenstrahlenbündel den Film nicht ganz deckt. Ausserdem führt das zu einer gewissen Unschärfe in der Aufnahme.

Der Erfindung liegt die Aufgabe zugrunde, ein Röntgenuntersuchungsgerät der eingangs genannten Art zu schaffen, bei dem bei einer Tomographie-Untersuchung der Röntgenzentralstrahl das Filmzentrum, unabhängig vom Objekt-Film-Abstand, immer trifft.

Diese Aufgabe ist erfindungsgemäss dadurch gelöst, dass die Anordnung aus zwei beweglichen, parallel zur Längsrichtung des Statives verlaufenden Zahnstangen besteht, in die im Achsbereich zwei starr miteinander verbundene, wahlweise gegenüber unterschiedlichen Teilen des Gerätes arretierbare Zahnräder bzw. Zahnradsegmente eingreifen, und in die im Bereich des Bildaufzeichnungsträgers zwei miteinander in Eingriff stehende Zahnräder eingreifen, mit denen der Bildaufzeichnungsträger zumindest indirekt verbunden ist, sowie mit Mitteln zur Verschiebung der Zahnräder entlang den Zahnstangen. Durch die Anordnung wird bei jeder Änderung des Film-Objekt-Abstandes der Bildaufzeichnungsträger derart entlang dem Stativ gefahren, dass der Film immer entlang dem Röntgenzentralstrahl bewegt wird.

In einer vorteilhaften Weiterbildung der Erfindung wird vorgeschlagen, dass die zwei starr miteinander verbundenen Zahnräder bzw. Zahnradsegmente raumfest arretierbar sind. Dadurch ist erreicht, dass eine Tomographie-Untersuchung aus einer beliebig gewinkelten Ausgangslage des Statives vorgenommen werden kann. Es hat den Vorteil, dass der Patient bei gewissen Untersuchungen nicht in unbequeme Lagen gebracht werden muss, sondern in Rückenbzw. Bauchlage bequem gelagert werden kann.

In einer weiteren Ausbildung der Erfindung wird vorgeschlagen, dass die zwei starr miteinander verbundenen Zahnräder bzw. Zahnradsegmente ortsfest am Stativ arretierbar sind. Dadurch ist erreicht, dass das Röntgenuntersuchungsgerät rasch von einem Tomographie-Untersuchungsgerat in ein konventionelles Röntgenuntersuchungsgerät, bei dem der Bildaufzeichnungsträger in jeder Lage des Statives senkrecht zum Röntgenzentralstrahl liegt, umgewandelt werden kann.

Weitere Vorteile und Einzelheiten der Erfindung ergeben sich aus den Unteransprüchen.

Die Erfindung ist nachfolgend anhand eines in den Zeichnungen dargestellten Ausführungsbeispieles näher erläutert. Es zeigen :

Fig. 1 eine schematische Darstellung eines Röntgenuntersuchungsgerätes mit einer Anordnung zum Verstellen eines Bildaufzeichnungs trägers nach der Erfindung,

Fig. 2 eine weitere Verstellmöglichkeit des Bildaufzeichnungsträgers mittels der Anordnung nach Fig. 1, und

Fig. 3 eine Perspektivansicht eines Röntgenuntersuchungsgerätes nach den Fig. 1 und 2.

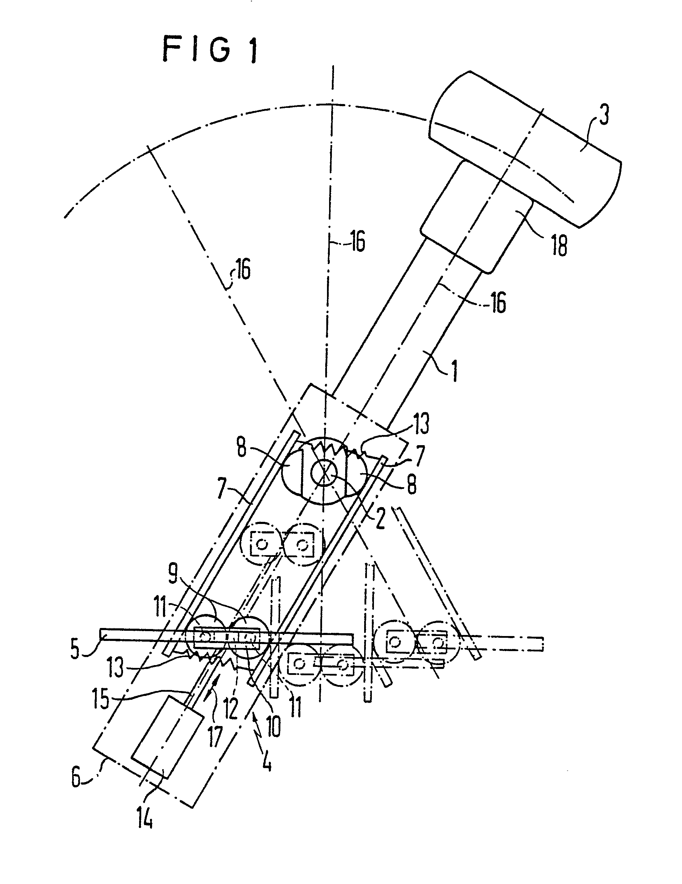

In den Figuren ist ein als Pendelstativ ausgebildetes Röntgenuntersuchungsgerät gezeigt, mit dem sowohl konventionelle Röntgenaufnahmen als auch Tomographie-Aufnahmen vorgenommen werden können. In der Figur 1 ist das Röntgenuntersuchungsgerät mit später beschriebenen Mitteln so eingestellt, dass Tomographie-Aufnahmen durchgeführt werden können. Das Röntgenuntersuchungsgerät weist ein Stativ 1 auf, das um eine Achse 2 geschwenkt wird. Das Stativ 1 ist auf der einen Seite der Achse 2 mit einer Röntgenröhre 3 und auf der anderen Seite mit einer Anordnung 4 zum Verstellen eines Bildaufzeichnungsträgers 5, z. B. eine Filmkassette, entlang dem Stativ 1 versehen.

Die Anordnung zum Verstellen der Filmkassette 5 entlang dem Stativ 1 besteht aus zwei in einem Gehäuse 6 angeordneten, parallel zur Längsrichtung des Statives 1 verlaufenden Zahnstangen 7.

Im Bereich der Achse 2 des Statives 1 greifen zwei starr miteinander verbundene Zahnradsegmente 8 ein, die wahlweise gegenüber unterschiedlichen Teilen des Gerätes arretierbar sind. In dem in Fig. 1 gezeigten Beispiel sind die Zahnradsegmente unabhängig von der Schwenkbewegung des Statives 1, d. h. raumfest mit bekannten und daher nicht dargestellten Mitteln arretiert. Zwischen den Zahnstangen 7 sind zwei miteinander und mit den Zahnstangen 7 in Eingriff stehende Zahnräder 9 angeordnet. Die Zahnräder 9 sind über eine Platte 10, die an den jeweiligen Achsen 11 der Zahnräder 9 angebracht ist, miteinander verbunden. Die Kassettenhalterung 5 ist über eine Halterung 12 an der Platte 10 befestigt. Ferner sind die Zahnstangen 7 mittels Federn 13 elastisch miteinander verbunden.

Zum Verschieben der Kassettenhalterung 5 entlang dem Stativ 1 ist ein elektrischer Motor 14 vorgesehen, der eine Stange 15, deren freies Ende an der Platte 10 befestigt ist, in Richtung der Pfeile 17 steuert.

Bei einer Tomographie-Untersuchung liegt der aufzunehmende Bereich eines nicht dargestellten Patienten bzw. die Objektebene in der Achse 2 des Statives 1. Das Objekt wird aufgenommen, während das Stativ 1 mit der Röntgenröhre 3 um die Achse 2 bis zu vorbestimmten Winkellagen geschwenkt wird. In der Fig. 1 sind verschiedene Winkellagen des Röntgenzentrahlstrahles 16 und ein Teil der Anordnung 4 mit der Kassettenhalterung 5 bei einer Schwenkung des Statives 1 punktgestrichelt angedeutet. Die Anordnung 4 funktioniert wie ein Parallelogrammsystem, bei dem die Zahnstangen 7 beim Schwenken des Statives 1 um die Achse 2 auf dem jeweils zugeordneten Zahnsegment 8 rollen. In gleicher Weise rollen die Zahnstangen 7 auf den mit diesen im Eingriff stehenden Zahnrädern 9 derart, dass die Zahnräder 9 zusammen mit der Kassettenhalterung 5 eine im Raum parallele Bewegung mit dem gleichen Abstand zur Achse 2 beschreiben.

Wenn der Objekt-Film-Abstand verringert werden soll, werden die Zahnräder mittels der Stange 15 entlang den Zahnstangen geführt, bis der gewünschte Abstand erreicht worden ist. Dabei wird die Kassettenhalterung 5 entlang dem Stativ 1 derart verschoben, dass der Zentralstrahl 16 der Röntgenröhre 3 immer in der Mitte der Kassettenhalterung 5 und die Kassettenhalterung 5 im Vergleich zu ihrer ursprünglichen Lage immer parallel liegt. Auf diese Weise wird das durch die Röntgenröhre 3 erzeugte und von einer Blende 18 begrenzte Röntgenstrahlenbündel den Film in jeder Lage decken. Durch die in einer gewünschten Lage dreh- und arretierbaren Zahnsegmente 8 kann eine Tomographie-Untersuchung von einer beliebigen Winkellage ausgehen. Dies hat den Vorteil, dass der Patient bei einer derartigen Untersuchung immer in einer für ihn bequemen Lage gelagert werden kann.

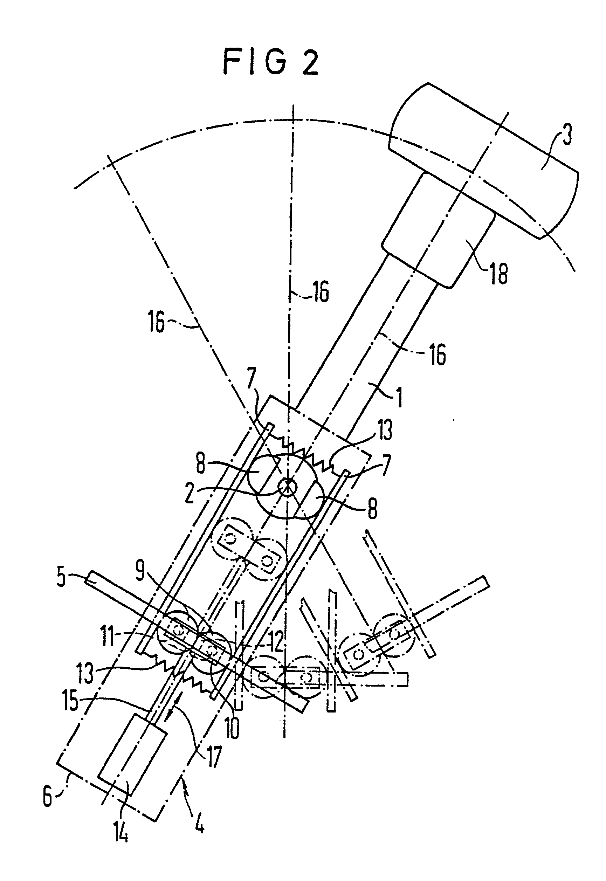

In der Figur 2 ist gezeigt, dass auch Röntgenaufnahmen in jeder Winkellage des Statives mit der Kassettenhalterung 5 immer senkrecht zum Röntgenzentralstrahl 16 vorgenommen werden können. Dies erfolgt, indem die Zahnradsegmente 8 ortsfest am Stativ 1 arretiert worden sind. Bei einer Schwenkbewegung des Statives 1 um die Achse 2 behalten die Teile 7 bis 13 der Anordnung 4 in jeder Winkellage des Statives in Beziehung zueinander ihre Lagen. Dies geht aus den punktgestrichelten Teilen der Anordnung und Kassettenhalterung hervor. Die Kassettenhalterung 5 kann auch in dieser Position in beschriebener Weise mittels des Motors 14 und der Stange 15 entlang dem Stativ 1 verschoben werden.

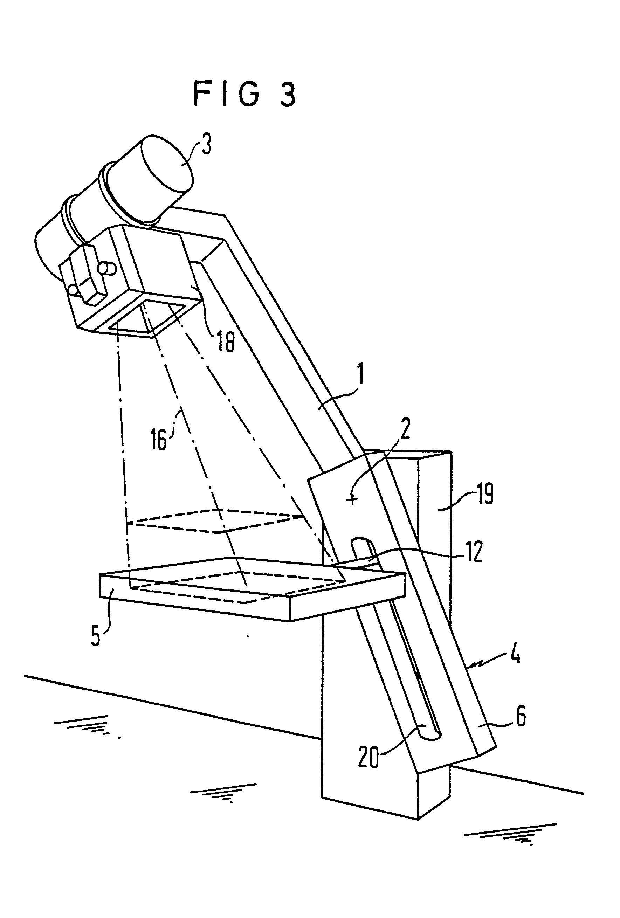

Das Röntgenuntersuchungsgerät kann ein Deckenstativ oder auch ein über eine Säule 19 auf dem Boden ruhendes Stativ sein, wie es in der Fig. 3 gezeigt ist. In dieser Figur, in der die Kassettenhalterung für eine Tomographie-Aufnahme eingestellt ist, ist dargestellt, dass die Halterung 12 der Kassettenhalterung 5 in einer im Gehäuse 6 verlaufenden Spur 20 läuft.

**Patentansprüche**

1. Röntgenuntersuchungsgerät mit einem um eine Achse schwenkbaren Stativ, das auf einer Seite der Achse mit einer Röntgenröhre und auf der anderen Seite mit einer Anordnung zum Verstellen eines Bildaufzeichnungsträgers entlang dem Stativ versehen ist, dadurch gekennzeichnet, dass die Anordnung (4) aus zwei beweglichen, parallel zur Längsrichtung des Statives (1) verlaufenden Zahnstangen (7) besteht, in die im Achsbereich (2) zwei starr mitelnander verbundene, wahlweise gegenüber unterschiedlichen Teilen des Gerätes arretierbare, Zahnräder bzw. Zahnradsegmente (8) eingreifen und in die im Bereich des Bildaufzeichnungsträgers (5) zwei miteinander in Eingriff stehende Zahnräder (9) eingreifen, mit denen der Bildaufzeichnungsträger (5) zumindest indirekt verbunden ist, sowie mit Mitteln (14, 15) zur Verschiebung der Zahnräder entlang den Zahnstangen.

2. Röntgenuntersuchungsgerät nach Anspruch 1, dadurch gekennzeichnet, dass die zwei starr miteinander verbundenen Zahnräder bzw. Zahnradsegmente (8) raumfest arretierbar sind.

3. Röntgenuntersuchungsgerät nach Anspruch 1, dadurch gekennzeichnet, dass die zwei starr miteinander verbundenen Zahnräder bzw. Zahnradsegmente (8) ortsfest am Stativ (1) arretierbar sind.

4. Röntgenuntersuchungsgerät nach einem der Ansprüche 1 bis 3, dadurch gekennzeichnet, dass die Zahnstangen (7) elastisch miteinander verbunden sind.

5. Röntgenuntersuchungsgerät nach einem der Ansprüche 1 bis 4, dadurch gekennzeichnet, dass die Zahnräder (9) über mindestens eine Platte (10), die an den jeweiligen Achsen (11) der Zahnräder (9) angebracht ist, miteinander verbunden sind.

6. Röntgenuntersuchungsgerät nach Anspruch 5, dadurch gekennzeichnet, dass der Bildaufzeichnungsträger (5) über eine Halterung (12) mit der Platte (10) verbunden ist.

7. Röntgenuntersuchungsgerät nach einem der Ansprüche 1 bis 6, dadurch gekennzeichnet dass als Mittel zur Verschiebung eine mechanische Kraftquelle (14) und eine die Kraft übertragende Stange (15) vorgesehen sind, die zumindest an einem Zahnrad (9) angreift.

## Claims

1. An X-ray examination device comprising a stand pivotable about an axis and provided with an X-ray tube on side of the axis, and with an arrangement for adjusting an image recording carrier along the stand on the other side of the axis, characterised in that the arrangement (4) consists of two mobile gear racks (7) which extend parallel to the longitudinal direction of the stand (1), and into which there engage, in the axial zone (2), two rigidly-interconnected gear wheels or gear wheel segments (8), which can be selectively locked in relation to different parts of the device, and into which two mutually-engaging gear wheels (9) engage, in the region of the image recording carrier (5), to which gear wheels (9) the image recording carrier (5) is connected, at least indirectly, and means (14, 15) being provided for moving the gear wheels along the gear racks.

2. An X-ray examination device as claimed in Claim 1, characterised in that the two rigidly-interconnected gear wheels or gear wheel segments (8) can be locked so as to be immobile.

3. An X-ray examination device as claimed in Claim 1, characterised in that the two rigidly-interconnected gear wheels or gear wheel segments (8) can be locked to the stand (1) in a fixed position.

4. An X-ray examination device as claimed in one of Claims 1 to 3, characterised in that the gear racks (7) are flexibly connected to one another.

5. An X-ray examination device as claimed in one of Claims 1 to 4, chacterised in that the gear wheels (9) are connected to one another via at least one plate (10) which bears a fixed relationship to the respective axes (11) of the gear wheels (9).

6. An X-ray examination device as claimed in Claim 5, characterised in that the image recording carrier (5) is connected to the plate (10) via a holder (12).

7. An X-ray examination device as claimed in one of Claims 1 to 6, characterised in that the displacement means consist of a mechanical power source (14) which transmits power via a rod (15) to act on at least one gear wheel (9).

## Revendications

1. Appareil de radiodiagnostic comportant un statif qui peut tourner autour d'un axe et comporte, d'un côté de l'axe, un tube à rayons X et, de l'autre côté, un dispositif servant à déplacer un support d'enregistrement d'images le long du statif, caractérisé par le fait que le dispositif (4) est constitué par deux crémaillères mobiles (7), qui sont parallèles à la direction longitudinale du statif (1) et avec lesquelles engrènent, dans la zone (2) de l'axe, deux pignons ou secteurs dentés (8) reliés rigidement entre eux et pouvant être bloqués au choix par rapport à différentes parties de l'appareil, et avec lesquels engrènent, au voisinage du support d'enregistrement d'images (5), deux pignons (9) réciproquement en prise et auxquels le support d'enregistrement d'images (5) est relié, au moins de façon indirecte, ainsi que des moyens (14, 15) servant à déplacer les pignons le long des crémaillères.

2. Appareil de radiodiagnostic suivant la revendication 1, caractérisé par le fait que les deux pignons ou secteurs dentés (8), reliés rigidement entre eux, peuvent être bloqués dans une position fixe dans l'espace.

3. Appareil de radiodiagnostic suivant la revendication 1, caractérisé par le fait que les deux pignons ou secteurs dentés (8), reliés rigidement entre eux, peuvent être bloqués d'une manière fixe sur le statif (1).

4. Appareil de radiodiagnostic suivant l'une des revendications 1 à 3, caractérisé par le fait que les crémaillères (7) sont reliées entre elles élastiquement.

5. Appareil de radiodiagnostic suivant l'une des revendications 1 à 4, caractérisé par le fait que les pignons (9) sont reliés entre eux par l'intermédiaire d'au moins une plaque (10), qui est montée sur les axes respectifs (11) des pignons (9).

6. Appareil de radiodiagnostic suivant la revendication 5, caractérisé par le fait que le support d'enregistrement d'images (5) est relié par l'intermédiaire d'un dispositif de retenue (12) à la plaque (10).

7. Appareil de radiodiagnostic suivant l'une des revendications 1 à 6, caractérisé par le fait qu'il est prévu, comme moyens de déplacement, une source de force mécanique (14) et une barre (15) transmettant la force et engrenant au moins avec un pignon (9).

# FIG 1

# FIG 2

# FIG 3